# EUROPÄISCHE PATENTSCHRIFT

(11) **EP 1 133 267 B1**
(45) Veröffentlichungstag und Bekanntmachung des Hinweises auf die Patenterteilung: **23.04.2003**
(21) Anmeldenummer: 99963319.1
(22) Anmeldetag: 20.11.1999
(51) Int. Cl.: A61F 2/36, A61F 2/38, A61F 2/30

(54) **ARMIERUNGSENDOPROTHESE**
REINFORCEMENT ENDOPROSTHESIS
ENDOPROTHESE D'ARMATURE

(30) Priorität: 26.11.1998 DE 19854517; 03.02.1999 DE 19904214
(43) Veröffentlichungstag der Anmeldung: 19.09.2001
(73) Patentinhaber: ESKA Implants GmbH & Co., 23556 Lübeck (DE)
(72) Erfinder: GRUNDEI, Hans, D-23558 Lübeck (DE); ASCHERL, Rudolf, D-85049 Ingolstadt (DE)
(74) Vertreter: Fuchs Mehler Weiss & Fritzsche
(86) Internationale Anmeldenummer: EP9908973
(87) Internationale Veröffentlichungsnummer: WO00030569

(56) Entgegenhaltungen:
- EP-A- 0 290 767
- DE-A- 3 138 848
- DE-A- 3 336 004
- DE-A- 3 535 158
- DE-A- 3 903 438
- DE-A- 4 039 064
- FR-A- 2 585 945
- GB-A- 2 070 939
- US-A- 4 384 373

## Beschreibung

Die vorliegende Erfindung betrifft eine Armierungsendoprothese zum Einsatz in einen Femurknochen, der sowohl im Bereich der Gelenkkugel des natürlichen Hüftgelenkes als auch im Bereich der Kondylen des natürlichen Kniegelenkes reseziert ist. Dementsprechend verfügt die Armierungsendoprothese über ein Stielteil in Bereichen des Hüftgelenkes sowie über ein Kondylenteil im Bereich des Kniegelenkes.

Es gibt Indikationen, bei denen die Kortikalis des Femurknochens entlastet werden muß, da zum Indikationszeitpunkt Krankheitsbilder wie Arthritis, Rheuma, Arthrose im Knie oder Osteoporose im Hüftgelenksbereich oder ein Trauma im Hüft- oder Kniegelenksbereich einen chirurgischen Eingriff erforderlich erscheinen lassen, gleichwohl nach der Therapie Hoffnung besteht, daß die Femur-Kortikalis ihre natürliche Funktion nach einer gewissen Zeit wieder wahrnehmen kann.

Eine Tumorendoprothese, wie beispielsweise gemäß der DE-A-40 39 064, vermag diese nur temporäre Entlastungsfunktion der anvisierten Armierungsendoprothese nicht zu leisten, da der Femurknochen bei der Implantation dieser Tumorendoprothese vollständig, da tumorbefallen, entfernt werden muß. Für die speziellen, oben beispielhaft genannten Krankheitsbilder eignet sich eine derartige Endoprothese nicht.

Eine andere Tumorendoprothese zeigt die US-A-4 384 373. Hier werden bedingt durch den Tumorbefall entsprechend große Knochenbereiche reseziert, welche dann nicht mehr zu Ablastfunktionen fähig ist. Gleichwohl ist man bemüht, dem Patienten so viel natürliches Knochenmaterial wie möglich zu belassen. Aus diesem Grunde ist in der Druckschrift vorgeschlagen worden, die Tumorprothese intramedullär einzusetzen, wobei die Prothese sowohl über ein Kondylenteil im Bereich des Kniegelenks als auch über eine den Schenkelhals sowie weitere Bereiche des natürlichen Femurknochens nachbildende Überbrückung verfügt, an deren Ende schließlich die künstliche Gelenkkugel des Hüftgelenks sitzt. Indikationsbedingt kann der Restknochen - wie bereits ausgeführt - nicht belastet werden. Ziel der Implantation einer derartigen Tumorendoprothese ist es, den Patienten so gut wie möglich mit einem Hilfsmittel zu versorgen, damit sich dieser auf möglichst natürliche Art und Weise fortbewegen kann. Eine derartige Tumorprothese nimmt demnach eine permanente Hilfsfunktion wahr.

Für die oben erwähnten Indikationen ist auch diese Prothese daher nicht geeignet. Hinsichtlich der konstruktiven Merkmale aber wird diese als gattungsgemäß angesehen. Demgemäß ist vorgesehen, daß das Verbindungsteil zwischen dem Stielteil und dem Kondylenteil durch einen intramedullär verlaufenden zweiteiligen Marknagel gebildet ist, wobei das eine Teil des Marknagels ein massiver Marknagel mit einem Gewindeansatz und einem daran angesetzten Gewinde und das andere Teil des Marknagels ein hohler Marknagel mit zum Gewinde des ersten Teils passenden Innengewinde ist, welches mit dem Gewinde verschraubbar ist, wodurch die Länge der Endoprothese einstellbar ist.

Vor diesem Hintergrund ist es nun die Aufgabe der vorliegenden Erfindung, eine Armierungsendoprothese für den Femurknochen anzugeben, welche eine temporäre Entlastungsfunktion ausübt und welche universell eingesetzt werden kann, also patientenindividuell aus Standardbauteilen erstellt werden kann, und welche die Rotationsstabilität der Endoprothese sichert.

Gelöst wird diese Aufgabe dadurch, daß das Verbindungsteil rotationsunsymmetrische Konturen aufweist.

Das Verbindungsteil arbeitet demnach nach dem bekannten Prinzip des Spindeltriebs. Der Operateur kann die Länge des Marknagels und damit des Verbindungsteils zwischen den künstlichen Gelenkteilen somit patientenindividuell einstellen und die Endoprothese herrichten. Die Endoprothese besteht aus Standardteilen, vor allem im Hinblick auf den massiven Marknagel und den hohlen Marknagel. An diese beiden Teile können dann das patientenindividuell auszuwählende Stielteil bzw. Kondylenteil angekoppelt werden. Das Stielteil kann bevorzugt an dem massiven Marknagel mittels einer Sicherungsschraube arretiert sein in Verbindung mit einem konischen Klemmsitz zwischen einem konischen Steckzapfen und einer konischen Steckhülse. Das Kondylenteil wird bevorzugt ebenfalls durch die Wirkung einer konischen Verklemmung zwischen einer konischen Steckhülse und einem konischen Steckzapfen am hohlen Marknagel gehalten, wobei vorteilhafterweise am Übergang vom Kondylenteil zum hohlen Marknagel eine Sechskantsicherungsschraube in einer entsprechenden Aufnahme liegt. Mit letzterer Maßnahme wird eine innere Rotationsstabilität erzielt.

Es besteht darüber hinaus die Möglichkeit, durch gezieltes Verkürzen der Armierungs-Endoprothese in bezug auf den Femurknochen dessen Kortikales unter Druckspannung zu setzen. Dies fördert den Heilungsprozess des Knochens.

Operativ geht man nun bei der Implantation der erfindungsgemäßen Armierungsendoprothese so vor, daß zunächst der Femur im Hüftgelenksbereich, und zwar im Gegensatz zu dem Vorschlag gemäß der bereits genannten US-A-4 384 373 lediglich unter Entfernung des Oberschenkelhalses mit dem Hüftgelenkkopf, sowie im Kniegelenksbereich reseziert wird. Sodann wird der massive Marknagelteil, der beispielsweise mit dem Stielteil geeigneter Größe, Formgebung, etc. verbunden ist, in den Markraum des Femurknochens gestoßen. Von der anderen Seite her wird der zweite, hohle Marknagel in den Markraum gedrängt, solange, bis das Gewinde im hohlen Marknagel auf das Gewinde des massiven Marknagels trifft. Durch
eine Schraubbewegung des ersten Marknagels gegenüber dem zweiten Marknagel wird nun die Armierungsendoprothese auf Länge eingestellt.

Vorteilhaft sind die Dimensionen so ausgelegt, daß die Länge des intramedullären Marknagels um bis zu 10 cm variierbar ist. Das bedeutet, daß von einer Normallage die Länge um ± 5 cm durch Verstellung des Spindeltriebes verändert werden kann.

Die Belastungen, die normalerweise von der natürlichen Femur-Kortikalis übertragen werden, werden nun teilweise durch die Armierungsendoprothese aufgefangen. Im Verlauf der Zeit nimmt die Entlastungsfunktion ab und die Kortikalis wird immer stärker belastet, bis die natürliche Belastung wieder eintreten kann. Diese Zeitspanne des Überganges der Belastung von der Endoprothese hin zu der Kortikalis kann beispielsweise durch Verabreichung von Medikamenten bei bestimmten Krankheitsbildern begleitet werden, dergestalt, daß die Kortikalis im Laufe der Zeit belastbarer wird.

Besonders vorteilhaft ist die Ausbildung der Endoprothese dergestalt, daß die Oberfläche des Stielteils und die Anlagefläche des Kondylenteils am resezierten Femurknochen zumindest teilweise mit einer dreidimensionalen offenmaschigen Raumnetzstruktur versehen ist. Die Raumnetzstruktur kommt dann an der im Femurknochen verbliebenen Spongiosa zu liegen und regt dort die Knochentrapekel zum Wachstum an. Nach einer gewissen Zeit ist das Implantat vollständig einorganisiert in den Femurknochen und bildet einen innigen Verbund mit diesem. Nach vollständigem Einwachsen der Endoprothese übernimmt der überbrückende Marknagel praktisch keinerlei Entlastungsfunktion mehr.

Bei Einsatz der Endoprothese bei Fraktur des Femur kommen oftmals Osteosyntheseplatten zum Einsatz, um die frakturierten Knochenteile aneinander zu fixieren und um die Frakturstelle während der Ausheilung mechanisch zu stabilisieren. Dies gelingt in der Praxis recht gut, leidet aber unter dem Nachteil, daß die Osteosyntheseplatten zumeist nicht symmetrisch um die Fraktur angesetzt werden können, so daß aus der daraus folgenden Unsymmetrie auch Unsymmetrien hinsichtlich der in die Kortikalis des Femurs eingeleiteten Kräfte resultieren. Bei Patienten, welche wie im vorliegenden Fall mit zwei künstlichen Gelenken versorgt werden sollen, also mit einem künstlichen Hüftgelenk und mit einem künstlichen Kniegelenk, stellt sich oftmals die Frage nach der mechanischen Stabilität des Implantates, insbesondere bei zusätzlicher Fraktur des Femurs. Derartige schwere Indikationen treten beispielsweise nach schweren Unfällen auf.

Die erfindungsgemäße Endoprothese leistet auch hier Abhilfe, da sie für eine intramedulläre Stützung bis zur letztendlichen Ausheilung der Fraktur sorgt und sofort belastbar ist.

Die Rotationsstabilität der Armierungsendoprothese ist dadurch gesichert, daß die in den Femur greifenden Teile rotationsunsymmetrische Konturen aufweisen.

Die Lagerhaltung in den Kliniken wird bei dem erfindungsgemäßen Aufbau der Endoprothese niedrig gehalten, da der Marknagel aus Standardteilen zusammengesetzt werden kann und nur mit den patientenindividuellen Stielund Kondylenteilen bestückt werden muß. Dementsprechend werden die Lagerkosten niedrig gehalten und es wird weniger Kapital gebunden.

Die Erfindung wird anhand eines Ausführungsbeispieles näher erläutert.

Mit 1 ist das Stielteil bezeichnet, welches im Hüftbereich in den reserzierten Femurknochen gesetzt wird. Das Stielteil 1 trägt die künstliche Gelenkkugel 12 des künstlichen Hüftgelenks. Das Stielteil ist vorwiegend in großen Bereichen mit einer dreidimensionalen offenmaschigen Raumnetzstruktur 8 belegt, in welche und durch welche hindurch Knochenmaterial wachsen kann zur innigen und permanenten Fixation des Stielteils 1 im Femur.

An das Stielteil 1 ist distal über eine konische Klemmverbindung zwischen einem am Stielteil 1 angeformten konischen Steckzapfen und einer konischen Klemmhülse, die in einem Adapterabschnitt des massiven Marknagels 4 vorgesehen ist, der Marknagel 4 angekoppelt. Die Verbindung wird gesichert durch eine Sicherungsschraube 9.

Dem massiven Marknagel angeformt ist ein Gewinde 5 in Form einer Gewindespindel. Die Gewindespindel 5 greift in ein Innengewinde 7 des hohlen Marknagels 6, derart, daß durch Schraubbewegungen die Länge des Verbindungsteils 3 aus den Marknägeln 4 und 6 eingestellt werden kann.

Dem zweiten, hohlen Marknagel 6 schließt sich ein Adapterabschnitt für das Kondylenteil 2 des künstlichen Kniegelenkes an. Zur Ankopplung des Kondylenteils 2 ist in dem Adapterabschnitt eine konische Klemmhülse eingebracht, welche mit einem konischen Zapfen, der Bestandteil des Kondylenteils 2 ist, zusammenarbeitet, derart, daß beide Teile mittels einer konischen Klemmverbindung arretiert werden können.

Zur Rotationssicherung ist im übrigen nur am Übergang vom Kondylenteil 2 zum Marknagel 6 eine Sechskantsicherungsschraube 10 vorgesehen, die in eine entsprechend ausgeformte Aufnahme zu liegen kommt. Hierdurch ist eine Rotation des Kondylenteils 2 gegenüber der restlichen Endoprothese ausgeschlossen.

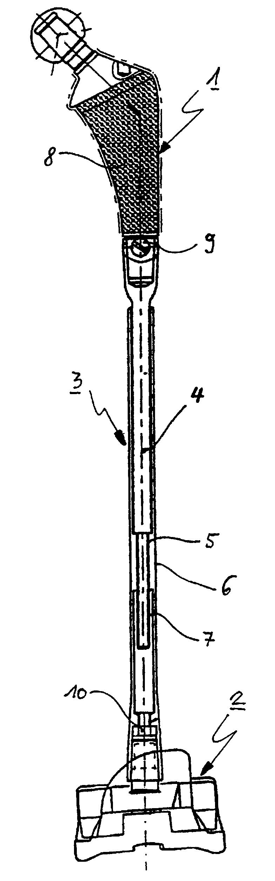

## Patentansprüche

1. Armierungsendoprothese zum Einsatz in einen sowohl hüftgelenkseitig als auch kniegelenkseitig resezierten Femurknochen mit einem Stielteil (1) eines künstlichen Hüftgelenks sowie mit einem Kondylenteil (2) eines künstlichen Kniegelenks, bei der das Verbindungsteil (3) zwischen dem Stielteil (1) und dem Kondylenteil (2) durch einen intramedullär verlaufenden zweiteiligen Marknagel gebildet ist, wobei das eine Teil des Marknagels ein massiver Marknagel (4) mit einem Gewindeansatz und einem daran angesetzten Gewinde (5) und das andere Teil des Marknagels ein hohler Marknagel (6) mit zum Gewinde (5) des ersten Teils passenden Innengewinde (7) ist, welches mit dem Gewinde (5) verschraubbar ist, wodurch die Länge der Endoprothese einstellbar ist,
**dadurch gekennzeichnet,**
**daß** das Verbindungsteil (3) rotationsunsymmetrische Konturen aufweist.

2. Armierungsendoprothese nach Anspruch 1, bei der die Länge des intramedullären Marknagels um bis zu 10 cm varüerbar ist.

3. Armierungsendoprothese nach Anspruch 1 oder 2, bei der die Oberfläche des Stielteils (1) und des Kondylenteils (2) zumindest teilweise mit einer dreidimensionalen, offenmaschigen Raumnetzstruktur (8) versehen ist.

4. Armierungsendoprothese nach einem der Ansprüche 1 bis 3, bei der das Stielteil (1) an dem massiven Marknagel (4) mittels einer Sicherungsschraube (9) arretiert ist.

5. Armierungsendoprothese nach einem der Ansprüche 1 bis 4, bei der am Übergang vom Kondylenteil (2) zum hohlen Marknagel (6) eine Sechskantsicherungsschraube (10) in einer entsprechenden Aufnahme (11) liegt.

## Claims

1. Reinforcing endoprosthesis for insertion in a femoral bone resected on the hip-joint side and on the knee-joint side, having a stem part (1) of an artificial hip joint as well as having a condyle part (2) of an artificial knee joint, in which the connecting part (3) is formed between the stem part (1) and the condyle part (2) by a two-part marrow pin running in intramedullary manner, wherein the one part of the marrow pin is a solid marrow pin (4) having a threaded attachment and a thread (5) attached thereto and the other part of the marrow pin is a hollow marrow pin (6) with internal thread (7) matching the thread (5) of the first part, which internal thread (7) can be screwed to the thread (5), as a result of which the length of the endoprosthesis can be adjusted, **characterised in that** the connecting part (3) has rotationally asymmetrical contours.

2. Reinforcing endoprosthesis according to claim 1, in which the length of the intramedullary marrow pin can be varied by up to 10 cm.

3. Reinforcing endoprosthesis according to claim 1 or 2, in which the surface of the stem part (1) and of the condyle part (2) is provided at least partly with a three-dimensional, open-meshed spatial network structure (8).

4. Reinforcing endoprosthesis according to one of claims 1 to 3, in which the stem part (1) is locked to the solid marrow pin (4) by means of a safety screw (9).

5. Reinforcing endoprosthesis according to one of claims 1 to 4, in which a hexagonal safety screw (10) lies in a corresponding recess (11) at the transition from the condyle part (2) to the hollow marrow pin (6).

## Revendications

1. Endoprothèse de consolidation destinée à être implantée dans un fémur, qui a subi une résection non seulement du côté de l'articulation de la hanche, mais aussi du côté de l'articulation du genou, comprenant une tige (1) d'une articulation artificielle de la hanche, ainsi qu'une partie formant le condyle (2) d'une articulation artificielle du genou, dans laquelle la partie de jonction (3) entre la tige (1) et la partie formant le condyle (2) est formée par un clou intramédullaire en deux parties implanté dans le canal médullaire, une partie du clou intramédullaire étant formée par un clou intramédullaire plein (4) muni d'une saillie filetée et d'un filet (5) réalisé sur celle-ci et l'autre partie du clou intramédullaire étant formée par un clou intramédullaire creux (6) muni d'un taraudage (7) adapté au filet (5) de la première partie et pouvant être vissé sur le filet (5), moyennant quoi il est possible de régler la longueur de l'endoprothèse,
**caractérisée en ce que**
la partie de jonction (3) possède des contours à dissymétrie de révolution.

2. Endoprothèse de consolidation selon la revendication 1, dans laquelle il est possible de faire varier jusqu'à 10 cm la longueur du clou intramédullaire.

3. Endoprothèse de consolidation selon la revendication 1 ou 2, dans laquelle la surface de la tige (1) et de la partie formant le condyle (2) est munie au moins en partie d'une structure réticulaire (8) tridimensionnelle à mailles ouvertes.

4. Endoprothèse de consolidation selon l'une quelconque des revendications 1 à 3, dans laquelle la tige (1) est bloquée sur le clou intramédullaire plein (4) au moyen d'une vis de blocage (9).

5. Endoprothèse de consolidation selon l'une quelconque des revendications 1 à 4, dans laquelle une vis de blocage à tête hexagonale (10) est vissée dans un logement (11) approprié dans la partie de transition entre la partie formant le condyle (2) et le clou intramédullaire creux (6).
